(19) Europäisches Patentamt
European Patent Office
Office européen des brevets

(11) EP 3 001 951 A1

(12) EUROPEAN PATENT APPLICATION
published in accordance with Art. 153(4) EPC

(43) Date of publication:
06.04.2016 Bulletin 2016/14

(51) Int Cl.:
A61B 5/1455 (2006.01)

(21) Application number: 13894519.1

(22) Date of filing: 23.10.2013

(86) International application number:
PCT/CN2013/085766

(87) International publication number:
WO 2015/043031 (02.04.2015 Gazette 2015/13)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 30.09.2013 CN 201310464640

(71) Applicant: Shenzhen Breo Technology Co., Ltd.
Shenzhen, Guangdong 518000 (CN)

(72) Inventors:
• MA, Xuejun
  Shenzhen
  Guangdong 518000 (CN)
• WU, Minghui
  Shenzhen
  Guangdong 518000 (CN)

(74) Representative: Krauns, Christian
Wallinger Ricker Schlotter Tostmann
Patent- und Rechtsanwälte
Zweibrückenstraße 5-7
80331 München (DE)

(54) METHOD AND SYSTEM FOR MEASURING OXYHEMOGLOBIN SATURATION IN BLOOD

(57) The present application relates to detection technology, it provides a detection method and detection system of blood oxygen saturation, this method includes: controlling two parallel light emitting diodes to emit a red light and an infrared light periodically to a finger; detecting the red light and infrared light passing through the finger and converting them into a red light electrical signal and an infrared light electrical signal respectively; separating pulsating components from the two electrical signals to obtain a separated variation of red light in one cycle and a separated variation of infrared light in one cycle; calculating the blood oxygen saturation based on the variations of red light and infrared light; amplifying and filtering the blood oxygen saturation prior to converting the blood oxygen saturation into digital signal by analog-to-digital converter.

controlling two parallel light emitting diodes to emit a red light and an infrared light periodically to a finger — 101

↓

detecting red and infrared lights and converting them into red light and infrared light electrical signals — 102

↓

separating pulsating components from two electrical signals to obtain variations of red light and infrared light — 103

↓

calculating blood oxygen saturation based on the variations of red light and infrared light — 104

↓

amplifying and filtering blood oxygen saturation then converting it into a digital signal by AD converter — 105

**Figure 1**

EP 3 001 951 A1

**Description**

Technical Field

The present application relates to the field of detection technology, in particular, it is directed to a method for detecting blood oxygen saturation and a system for detecting blood oxygen saturation.

Background

Oxygen content: the amount of oxygen carried by the hemoglobin in 100 ml of blood. The major portion is the oxygen bound to hemoglobin, and there is very small amount of oxygen dissolved in the blood plasma (only 0.3 ml %). After binding oxygen the hemoglobin becomes oxyhemoglobin ($HbO_2$), and after the oxygen is released it becomes reduced hemoglobin. The blood oxygen saturation ($SaO_2$) refers to the percentage of the actual oxygen combined (by hemoglobin) in the blood (oxygen content) in the maximum amount of the oxygen can be combined (by the hemoglobin) in the blood (oxygen capacity), therefore the blood oxygen saturation can be defined as follows:

$$SaO_2 = C_{Hb}O_2/(C_{Hb}O_2 + C_{Hb}) \times 100\%.$$

Blood oxygen saturation: the maximum amount of oxygen can be bound by the hemoglobin in 100 ml blood should be deemed as the oxygen capacity of the blood. The amount of oxygen binding to the hemoglobin is named as oxygen content, and the percentage of the oxygen content in the oxygen capacity is referred as blood oxygen saturation.
In the process of carrying out the exiting techniques, there are following technical problems in the prior art:

The existing method for detecting blood oxygen saturation is carried out in an invasive way, and this method has low efficiency and is harmful to the subject.

Summary

The aim of the embodiments according the present application is to provide a method for detecting the blood oxygen saturation in the blood, so as to solve such technical problems as low detection efficiency, and causing damage to the user.
The embodiments of the present application are carried out as follows: a method for detecting oxygen saturation in the blood comprising:

controlling two parallel light emitting diode to emit a red light and an infrared light periodically to a finger to be detected;
detecting the red light and infrared light passing through the finger, and converting the red light and infrared light passing through the finger into a red light electrical signal and infrared light electrical signal respectively;
separating the pulsating components from the two electrical signals to obtain a separated variation of red light in one cycle and a separated variation of infrared light in one cycle;
calculating the blood oxygen saturation based on the variations of red light and infrared light;
amplifying and filtering the blood oxygen saturation prior to converting the blood oxygen saturation into a digital signal and showing the result by means of an analog-to-digital converter (AD convertor).

Alternatively, the above step of calculating the blood oxygen saturation based on the variations of red light and infrared light may particularly comprise:

$$SaO_2 = \frac{1}{K} \, ln \, \frac{\triangle RED - \triangle N}{(\triangle IR - \triangle N) + \triangle RED - \triangle N} \times 100\%$$

Formula 3;

wherein $SaO_2$ is the blood oxygen saturation, K is the light absorption coefficient of the blood, $\triangle RED$ is the variation of the red light in one cycle, $\triangle IR$ is the variation of the infrared light in one cycle, $\triangle N$ is the disturbance value of the ambient light.

**EP 3 001 951 A1**

Alternatively, after the step of amplifying and filtering the blood oxygen saturation and prior to converting the blood oxygen saturation into a digital signal and showing the result by means of an analog-to-digital converter, the above method may further comprise:

calculating the pulse P according to the electrical signals of red light and infrared light; in particular,

$$ P = \frac{k^{IR}_{max} * C^{IR}_{max}}{k^{IR}_{min} * C^{IR}_{min}} \Big/ \frac{k^{R}_{max} * C^{R}_{max}}{k^{R}_{min} * C^{R}_{min}} \quad \text{Formula 4;} $$

wherein $k^{IR}_{max}$ is the maximum value of the absorption coefficient of the infrared light, $k^{IR}_{min}$ is the minimum value of the absorption coefficient of the infrared light;

$k^{R}_{max}$ is the maximum value of the absorption coefficient of the red light, and $k^{R}_{min}$ is the minimum value of the absorption coefficient of the red light;

$C^{IR}_{max}$ is the maximum value of the $C_{Hb}O_2$ concentration for infrared light, $C^{IR}_{min}$ is the minimum value of the $C_{Hb}O_2$ concentration for infrared light;

$C^{R}_{max}$ is the maximum value of the $C_{Hb}O_2$ concentration for red light, $C^{R}_{min}$ is the minimum value of the $C_{Hb}O_2$ concentration for red light.

On the other hand, a measurement system of oxygen saturation in the blood is provided, which comprises:

a control unit for controlling two parallel light emitting diode to emit a red light and infrared light periodically to a finger to be detected;
a detection unit for detecting the red light and infrared light passing through the finger, and converting the red light and infrared light passing through the finger into a red light electrical signal and an infrared light electrical signal;
a separation unit for separating the pulsating components from the two electrical signals respectively to obtain a separated variation of red light in one cycle and a separated variation of infrared light in one cycle;
a calculation unit for calculating the blood oxygen saturation according to the variations of red light and infrared light;
a conversion and transmission unit for converting the blood oxygen saturation into a digital signal and showing the result by means of an analog-to-digital converter after amplifying and filtering of the blood oxygen saturation.

Alternatively, the calculation unit is particularly used for

$$ SaO_2 = \frac{1}{K} \ln \frac{\triangle RED - \triangle N}{(\triangle IR - \triangle N) + \triangle RED - \triangle N} \times 100\% $$

Formula 3;

wherein $SaO_2$ is the blood oxygen saturation, K is the light absorption coefficient of the blood, $\triangle RED$ is the variation of the red light in one cycle, $\triangle IR$ is the variation of the infrared light in one cycle, $\triangle N$ is the disturbance value of the ambient light.
Alternatively, the calculation unit can be further used for calculating the pulse P based on the electrical signals of red light and infrared light; in particular,

3

$$P = \frac{k_{\max}^{IR} * C_{\max}^{IR}}{k_{\min}^{IR} * C_{\min}^{IR}} \bigg/ \frac{k_{\max}^{R} * C_{\max}^{R}}{k_{\min}^{R} * C_{\min}^{R}} \qquad \text{Formula 4;}$$

wherein $k_{\max}^{IR}$ is the maximum value of the absorption coefficient of the infrared light, and $k_{\min}^{IR}$ is the minimum value of the absorption coefficient of the infrared light;

$k_{\max}^{R}$ is the maximum value of the absorption coefficient of the red light, and $k_{\min}^{R}$ is the minimum value of the absorption coefficient of the red light;

$C_{\max}^{IR}$ is the maximum value of the $C_{Hb}O_2$ concentration for infrared light, and $C_{\min}^{IR}$ is the minimum value of the $C_{Hb}O_2$ concentration for infrared light;

$C_{\max}^{R}$ is the maximum value of the $C_{Hb}O_2$ concentration for red light, and $C_{\min}^{R}$ is the minimum value of the $C_{Hb}O_2$ concentration for red light;

Compared with the technical solutions in prior art, the embodiments of the present application have the following advantages: the oxygen content in the blood can be detected by means of the present technical solution in a noninvasive and real time way, therefore it has the advantages of noninvasive and real time measurement.

Brief Description of the Drawings

Figure 1 is flow chart of detection method of the blood oxygen saturation according to an embodiment of the present application.
Figure 2 is a schematic structural diagram of the measurement system of blood oxygen saturation according to the present application.

Detailed Description of the Invention

Objects, technical solution and advantages of the present application will be explained below in detail with reference to the accompanying drawings and examples. However, it should be appreciated that the following description of the embodiments is merely exemplary in nature and is in no way intended to limit the application.

The principle of the present application is as follows: the method according to the present disclosure is similar to the measurement method of spectrophotometric technology, the present method is based on the absorption spectrum characteristics of oxyhemoglobin ($HbO_2$) and reduced hemoglobin (Hb). When a beam of light shoots into a solution of a substance, the transmitted light intensity (I) and the emitted light intensity ($I_0$) satisfy the following relationship:

$$I = I_0 e^{KCd} \qquad \text{Formula 1}$$

wherein the natural logarithm of the ratio value of I to $I_0$ is named as light density D, accordingly formula 1 can also be expressed as:

$$D = \ln(I/I_0) = kCd; \qquad \text{Formula 2}$$

wherein C is the concentration of a solution (such as blood), d refers to the path of light passing through the blood, k is the light absorption coefficient of the blood. If the path d keeps constant, the concentration of blood will be proportional to the light density D. $HbO_2$ and Hb in blood have different absorption coefficients to lights with different wave length, it has been proved by experiments that, in the region of red light (RED) with 600 - 700 nm wave length, the absorption coefficient of Hb is much larger than that of $HbO_2$; while in the region of infrared light (IR) with 800 - 1000 nm wave length, the absorption coefficient of Hb is smaller than that of $HbO_2$; and the isoabsorptive point is observed at the vicinity of 805 nm.

The blood in a human body flows through the lungs under the systole and diastole of the heart, and a certain amount of reduced hemoglobin (Hb) binds the oxygen taken from the alveoli of lung and turns into oxyhemoglobin ($HbO_2$), about 98% of the oxygen combines with the hemoglobin to form oxyhemoglobin then it entries the tissues. This amount of oxygen reaches the blood capillaries along the artery system, then the oxygen is released so as to maintain the metabolism of the organizations. In one cardiac cycle, the ventricular systole and diastole cause the cyclic pressure fluctuation in the artery which makes the artery expand and retract, as a result the artery vessels produce regular pulse.

The probe employed in the pulse blood oxygen device according to the present application is set on the finger in use. There are two parallel light emitting diodes (LED) fixed onto the upper wall, the LEDs emit a red light of 660 nm and an infrared light of 940 nm. There is a photodetector disposed on the lower wall, which converts the red light and the infrared light transmitting through the artery vessels of the finger into electrical signals, the weaker the detected photoelectric signal is, the more light has been absorbed by the tissues, bone and blood when the light signal passes through the probe place. Skin, muscle, fat, venous blood, pigment, bones and the like have constant absorption coefficients to these two types of light, respectively, therefore they only have an impact on the value of DC component in the photoelectric signal, but the concentrations of $HbO_2$ and Hb in the blood change periodically with the function of blood pulse, therefore the light absorption thereof varies in a pulse-like way, accordingly the signal strength output by the photodetector changes in a pulse-like way with variation of concentration ratio of $HbO_2$ to Hb in the blood. If denoted with light absorption, when the red light and infrared light play function, the variation laws thereof are substantially the same, but the amplitude of the pulsation component may be different from each other. The emitting sequence of the two LEDs can be controlled by a timing circuit, and the sequence can be: (1) red light LED turns on; (2) red light LED turns off, and infrared LED turns on; (3) both two LEDs turn off; this emitting sequence runs repeatedly in a frequency of 400 times per second (50 Hz AC), so the inhibition ability to the ambient light can be enhanced. The red light and infrared light with different wave lengths pass through the part to be detected in turns, and the pulsation components in these two signals are separated respectively, after amplifying and filtering, the pulsation components are converted into digital signal by an A/D convertor. The intensity of the received light varies with the pulse, and the maximum value in the transmitted light intensity received by a detector under red light wave length is recorded as IRmax, and the minimum value is IRmin. IRmax appears in the period of systole, which corresponds to the time of maximum blood capacity of the artery, IRmin appears in the period of diastole, which corresponds to the minimum blood capacity of the artery.

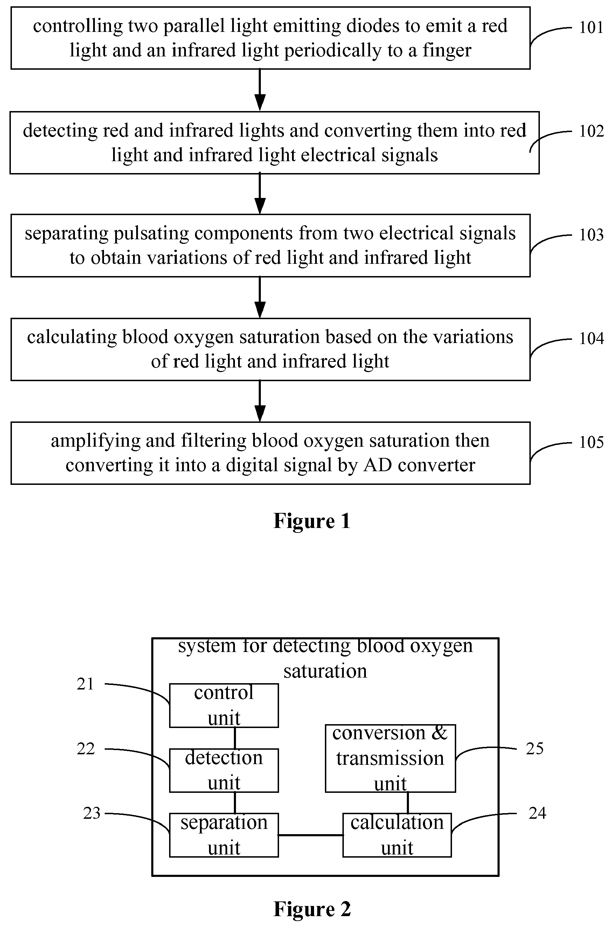

The embodiments of the present application provide a method for detecting the oxygen saturation in blood. The present method is carried out by a pulse blood oxygen device comprising: two light emitting diodes, and one photodetector, wherein one light emitting diode emits a red light of 660 nm wave length and the other emits an infrared light of 940 nm. The present method is shown in Figure 1, which comprises the following steps:

101: controlling two parallel light emitting diodes to emit red light and infrared light periodically to a finger to be detected;
102: detecting the red light and infrared light passing through the finger, and converting the transmitted red light and infrared light into a red light electrical signal and infrared light electrical signal;
103: separating the pulsating components from the two electrical signals to obtain a separated variation of red light in one cycle and a separated variation of infrared light in one cycle;
104: calculating the blood oxygen saturation based on the variations of red light and infrared light;
105: amplifying and filtering the blood oxygen saturation prior to converting the blood oxygen saturation into a digital signal and showing the result by means of an analog-to-digital converter (AD convertor).

Alternatively, the above cycle can be 0.02 second, i.e. the frequency thereof is 50 Hz AC frequency. Of course other frequencies can also be used for calculation.

The method for achieving the above step 104 can be:

$$SaO_2 = \frac{1}{K} \, ln \frac{\triangle RED - \triangle N}{(\triangle IR - \triangle N) + \triangle RED - \triangle N} \times 100\%$$

Formula 3;

wherein K is the light absorption coefficient of the blood, $_\triangle$RED is the variation of the red light in one cycle, $_\triangle$IR is the

variation of the infrared light in one cycle, $_\Delta$N is the disturbance value of the ambient light.

Alternatively, the above method can include the following step after step 105:

calculating the pulse P according to the electrical signals of red light and infrared light; in particular,

$$P = \frac{k_{max}^{IR} * C_{max}^{IR}}{k_{min}^{IR} * C_{min}^{IR}} \Big/ \frac{k_{max}^{R} * C_{max}^{R}}{k_{min}^{R} * C_{min}^{R}} \quad \text{Formula 4;}$$

wherein $k_{max}^{IR}$ is the maximum value of the absorption coefficient of the infrared light, and $k_{min}^{IR}$ is the minimum value of the absorption coefficient of the infrared light;

$k_{max}^{R}$ is the maximum value of the absorption coefficient of the red light, and $k_{min}^{R}$ is the minimum value of the absorption coefficient of the red light;

$C_{max}^{IR}$ is the maximum value of the $C_{Hb}O_2$ concentration for infrared light, and $C_{min}^{IR}$ is the minimum value of the $C_{Hb}O_2$ concentration for infrared light;

$C_{max}^{R}$ is the maximum value of the $C_{Hb}O_2$ concentration for red light, and $C_{min}^{R}$ is the minimum value of the $C_{Hb}O_2$ concentration for red light.

The embodiments according to the present application have the advantages of non-invasive real-time for detection of the blood oxygen saturation and pulse.

The embodiments of the present application further provide a system for detecting the oxygen saturation in blood as shown in Figure 2, wherein the system comprises:

a control unit 21 for controlling two parallel light emitting diode to emit a red light and infrared light periodically to a finger to be detected;
a detection unit 22 for detecting the red light and infrared light passing through the finger and converting the red light and infrared light passing through the finger into a red light electrical signal and an infrared light electrical signal;
a separation unit 23 for separating the pulsating components from the two electrical signals to obtain a separated variation of red light in one cycle and a separated variation of infrared light in one cycle;
a calculation unit 24 for calculating the blood oxygen saturation according to the variations of red light and infrared light;
a conversion and transmission unit 25 for converting the blood oxygen saturation into a digital signal and showing the result by means of an analog-to-digital converter after amplifying and filtering of the blood oxygen saturation.

Alternatively, the calculation unit 24 is particularly used for

$$SaO_2 = \frac{1}{K} \ln \frac{_\Delta RED - _\Delta N}{(_\Delta IR - _\Delta N) + _\Delta RED - _\Delta N} \times 100\%$$

Formula 3;

wherein, $SaO_2$ is the blood oxygen saturation, K is the light absorption coefficient of the blood, $_\Delta$RED is the variation of the red light in one period, $_\Delta$IR is the variation of the infrared light in one cycle, $_\Delta$N is the disturbance value of the ambient light.

Alternatively, the above calculation unit 24 can be further used for calculating the pulse P according to the electrical signals of red light and infrared light; in particular,

$$P = \frac{k_{max}^{IR} * C_{max}^{IR}}{k_{min}^{IR} * C_{min}^{IR}} \Big/ \frac{k_{max}^{R} * C_{max}^{R}}{k_{min}^{R} * C_{min}^{R}} \quad \text{Formula 4;}$$

wherein $k_{max}^{IR}$ is the maximum value of the absorption coefficient of the infrared light, and $k_{min}^{IR}$ is the minimum value of the absorption coefficient of the infrared light;

$k_{max}^{R}$ is the maximum value of the absorption coefficient of the red light, and $k_{min}^{R}$ is the minimum value of the absorption coefficient of the red light;

$C_{max}^{IR}$ is the maximum value of the $C_{Hb}O_2$ concentration for infrared light, and $C_{min}^{IR}$ is the minimum value of the $C_{Hb}O_2$ concentration for infrared light;

$C_{max}^{R}$ is the maximum value of the $C_{Hb}O_2$ concentration for red light, and $C_{min}^{R}$ is the minimum value of the $C_{Hb}O_2$ concentration for red light.

It should be appreciated that, all the units in the above-mentioned embodiments are just divided by their functions, but it should not limited to the above-mentioned division, if only the corresponding function can be achieved. On the other hand, the specific name of each functional unit is merely used to distinguish each other, but not used to limit the protection scope of the present application.

In addition, one skilled in the art should appreciate all or a certain part of the steps in each of the above embodiments can be completed by programs instructing correlated hardware, wherein the corresponding programs can be stored in a computer-readable storage medium such as ROM/RAM, disk, Compact Disc or the like.

The embodiments above are merely the preferable embodiments of the present application and not intended to limit the present application. And all changes equivalent substitution and improvements which come within the meaning and range of equivalency of the present application are intended to be embraced therein.

## Claims

1. A method for detecting oxygen saturation in a blood comprising:

controlling two parallel light emitting diodes to respectively emit a red light and an infrared light periodically to a finger to be detected;
detecting the red light and infrared light passing through the finger, and converting the transmitted red light and infrared light into a red light electrical signal and an infrared light electrical signal respectively;
separating pulsating components from the two electrical signals to obtain a separated variation of red light in one cycle and a separated variation of infrared light in one cycle;
calculating the blood oxygen saturation based on the variations of red light and infrared light;
amplifying and filtering the blood oxygen saturation prior to converting the blood oxygen saturation into a digital signal by means of an analog-to-digital converter.

2. The method of claim 1, wherein the step of calculating the blood oxygen saturation based on the variations of red light and infrared light comprises:

$$SaO_2 = \frac{1}{K} \ln \frac{\triangle RED - \triangle N}{(\triangle IR - \triangle N) + \triangle RED - \triangle N} \times 100\%$$

Formula 3;

wherein $SaO_2$ is the blood oxygen saturation, K is a light absorption coefficient of a blood, $_\triangle RED$ is a variation of the red light in one period, $_\triangle IR$ is a variation of the infrared light in one cycle, and $_\triangle N$ is a disturbance value of an ambient light.

3. The method of claim 1, wherein the step of amplifying and filtering the blood oxygen saturation prior to converting the blood oxygen saturation into a digital signal by means of an analog-to-digital converter further comprises:

calculating the pulse P according to the electrical signals of red light and infrared light; in particular,

$$P = \frac{k_{max}^{IR} * C_{max}^{IR}}{k_{min}^{IR} * C_{min}^{IR}} / \frac{k_{max}^{R} * C_{max}^{R}}{k_{min}^{R} * C_{min}^{R}} \quad \text{Formula 4,}$$

wherein $k_{max}^{IR}$ is the maximum value of an absorption coefficient of the infrared light, and $k_{min}^{IR}$ is the minimum value of an absorption coefficient of the infrared light;

$k_{max}^{R}$ is the maximum value of an absorption coefficient of the red light, and $k_{min}^{R}$ is the minimum value of an absorption coefficient of the red light;

$C_{max}^{IR}$ is the maximum value of a $C_{Hb}O_2$ concentration for infrared light, and $C_{min}^{IR}$ is the minimum value of a $C_{Hb}O_2$ concentration for infrared light;

$C_{max}^{R}$ is the maximum value of a $C_{Hb}O_2$ concentration for red light, and $C_{min}^{R}$ is the minimum value of a $C_{Hb}O_2$ concentration for red light.

4. A system for detecting oxygen saturation in a blood comprising:

a control unit for controlling two parallel light emitting diodes to respectively emit a red light and infrared light periodically to a finger to be detected;

a detection unit for detecting the red light and infrared light passing through the finger, and converting the transmitted red light and infrared light into a red light electrical signal and an infrared light electrical signal respectively;

a separation unit for separating pulsating components from the two electrical signals to obtain a separated variation of red light in one cycle and a separated variation of infrared light in one cycle;

a calculation unit for calculating the blood oxygen saturation according to the variations of red light and infrared light;

a conversion and transmission unit for converting the blood oxygen saturation into a digital signal by means of an analog-to-digital converter after amplifying and filtering of the blood oxygen saturation.

5. The system of claim 4, wherein the calculation unit is used for

$$SaO_2 = \frac{1}{K} \, ln \frac{_\triangle RED - _\triangle N}{(_\triangle IR - _\triangle N) + _\triangle RED - _\triangle N} \times 100\%$$

Formula 3;

wherein $SaO_2$ is the blood oxygen saturation, K is the light absorption coefficient of the blood, $_\triangle RED$ is a variation of the red light in one period, $_\triangle IR$ is a variation of the infrared light in one cycle, $_\triangle N$ is a disturbance value of an ambient light.

6. The system of claim 4, wherein the calculation unit can be further used for calculating the pulse P according to the electrical signals of red light and infrared light; in particular,

$$P = \frac{k^{IR}_{max} * C^{IR}_{max}}{k^{IR}_{min} * C^{IR}_{min}} / \frac{k^{R}_{max} * C^{R}_{max}}{k^{R}_{min} * C^{R}_{min}} \qquad \text{Formula 4;}$$

wherein $k^{IR}_{max}$ is the maximum value of an absorption coefficient of the infrared light, and $k^{IR}_{min}$ is the minimum value of an absorption coefficient of the infrared light;

$k^{R}_{max}$ is the maximum value of an absorption coefficient of the red light, and $k^{R}_{min}$ is the minimum value of an absorption coefficient of the red light;

$C^{IR}_{max}$ is the maximum value of a $C_{Hb}O_2$ concentration for infrared light, and $C^{IR}_{min}$ is the minimum value of a $C_{Hb}O_2$ concentration for infrared light;

$C^{R}_{max}$ is the maximum value of a $C_{Hb}O_2$ concentration for red light, and $C^{R}_{min}$ is the minimum value of a $C_{Hb}O_2$ concentration for red light.

controlling two parallel light emitting diodes to emit a red
light and an infrared light periodically to a finger — 101

detecting red and infrared lights and converting them into red
light and infrared light electrical signals — 102

separating pulsating components from two electrical signals
to obtain variations of red light and infrared light — 103

calculating blood oxygen saturation based on the variations
of red light and infrared light — 104

amplifying and filtering blood oxygen saturation then
converting it into a digital signal by AD converter — 105

**Figure 1**

system for detecting blood oxygen
saturation

21 — control
unit

22 — detection
unit

conversion &
transmission
unit — 25

23 — separation
unit

calculation
unit — 24

**Figure 2**

# INTERNATIONAL SEARCH REPORT

| International application No. |
| --- |
| **PCT/CN2013/085766** |

**A. CLASSIFICATION OF SUBJECT MATTER**

A61B 5/1455 (2006.01) i

According to International Patent Classification (IPC) or to both national classification and IPC

**B. FIELDS SEARCHED**

Minimum documentation searched (classification system followed by classification symbols)

A61B 5/145, 5/1455

Documentation searched other than minimum documentation to the extent that such documents are included in the fields searched

Electronic data base consulted during the international search (name of data base and, where practicable, search terms used)

WPI, EPDOC, CNPAT, CNKI: red light, infrared light, saturation, cycle?, puls+

**C. DOCUMENTS CONSIDERED TO BE RELEVANT**

| Category* | Citation of document, with indication, where appropriate, of the relevant passages | Relevant to claim No. |
| --- | --- | --- |
| Y | CN 101080192 A (KONINKLIJKE PHILIPS ELECTRONICS N.V.), 28 November 2007 (28.11.2007), description, page 3, line 26 to page 6, line 30, and figures 1-4 | 1, 4 |
| Y | US 4819646 A (PHYSIO-CONTROL CORPORATION), 11 April 1989 (11.04.1989), description, column 6, line 54 to column 15, line 24, abstract, and figures 1-11 | 1, 4 |
| A | CN 102846323 A (CHINA JILIANG UNIVERSITY), 02 January 2013 (02.01.2013), the whole document | 1-6 |
| A | CN 201175331 Y (CONTEC MEDICAL SYSTEMS CO., LTD.), 07 January 2009 (07.01.2009), the whole document | 1-6 |
| A | CN 201104882 Y (NEUSOFT MEDICAL SYSTEMS CO., LTD.), 27 August 2008 (27.08.2008), the whole document | 1-6 |
| A | US 5267562 A (NIHON KOHDEN CORPORATION), 07 December 1993 (07.12.1993), the whole document | 1-6 |

☐ Further documents are listed in the continuation of Box C.　　☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 27 June 2014 (27.06.2014) | **15 July 2014 (15.07.2014)** |

| Name and mailing address of the ISA/CN: State Intellectual Property Office of the P. R. China No. 6, Xitucheng Road, Jimenqiao Haidian District, Beijing 100088, China Facsimile No.: (86-10) 62019451 | Authorized officer **ZHENG, Qiwei** Telephone No.: (86-10) **62085633** |
| --- | --- |

Form PCT/ISA/210 (second sheet) (July 2009)

## INTERNATIONAL SEARCH REPORT
### Information on patent family members

| International application No. |
| --- |
| **PCT/CN2013/085766** |

| Patent Documents referred in the Report | Publication Date | Patent Family | Publication Date |
| --- | --- | --- | --- |
| CN 101080192 A | 28 November 2007 | WO 2006064399 A2 | 22 June 2006 |
| | | EP 1830695 A2 | 12 September 2007 |
| | | EP 1830695 B1 | 30 November 2011 |
| | | CN 101080192 B | 08 September 2010 |
| | | US 2009240125 A1 | 24 September 2009 |
| | | US 8315682 B2 | 20 November 2012 |
| | | AT 535184 T | 15 December 2011 |
| US 4819646 A | 1 1 April 1989 | AU 7718587 A | 25 February 1988 |
| | | WO 8801147 A1 | 25 February 1988 |
| | | EP 0261789 B1 | 31 March 1993 |
| | | JP H01502237 A | 10 August 1989 |
| | | US 4892101 A | 09 January 1990 |
| | | AU 606830 B2 | 14 February 1991 |
| | | CA 1300397 C | 12 May 1992 |
| | | AT 87449 T | 15 April 1993 |
| | | DE 3785123 T2 | 30 September 1993 |
| CN 102846323 A | 02 January 2013 | None | |
| CN 201175331 Y | 07 January 2009 | None | |
| CN 201104882 Y | 27 August 2008 | None | |
| US 5267562 A | 07 December 1993 | JP H06174 A | 11 January 1994 |
| | | JP 3091929 B | 25 September 2000 |

Form PCT/ISA/210 (patent family annex) (July 2009)